# EUROPEAN PATENT SPECIFICATION

(11) **EP 3 639 035 B1**
(45) Date of publication and mention of the grant of the patent: **21.06.2023**
(21) Application number: 18729158.8
(22) Date of filing: 12.06.2018
(51) Int. Cl.: G01N 33/68

(54) **FATTY ACID BINDING PROTEIN 3 FOR THE ASSESSMENT OF ATRIAL FIBRILLATION (AF)**
FETTSÄUREBINDENDES PROTEIN 3 ZUR BEURTEILUNG VON VORHOFFLIMMERN (VF)
PROTÉINE 3 LIANT LES ACIDES GRAS POUR L'ÉVALUATION DE LA FIBRILLATION AURICULAIRE (AF)

(30) Priority: 13.06.2017 EP 17175672
(43) Date of publication of application: 22.04.2020
(73) Proprietor: Roche Diagnostics GmbH, 68305 Mannheim (DE); F. Hoffmann-La Roche AG, 4070 Basel (CH); Maastricht University Medical Center, 6229 GT Maastricht (NL)
(72) Inventor: KARL, Johann, 82377 Penzberg (DE); KASTNER, Peter, 82377 Penzberg (DE); ROLNY, Vinzent, 82377 Penzberg (DE); WIENHUES-THELEN, Ursula-Henrike, 82377 Penzberg (DE); DIETRICH, Manuel, 82377 Penzberg (DE); ZIEGLER, André, 6343 Rotkreuz (CH); SCHOTTEN, Uli, 52076 Aachen (DE)
(74) Representative: Altmann Stößel Dick Patentanwälte PartG mbB
(86) International application number: PCT/EP2018/065503
(87) International publication number: WO 2018/229051

(56) References cited:
- WO-A1-2016/012783
- US-A1- 2011 072 892
- YOICHIRO OTAKI ET AL: "Prognostic Value of Myocardial Damage Markers in Patients with Chronic Heart Failure with Atrial Fibrillation", INTERNAL MEDICINE., vol. 53, no. 7, 1 January 2014 (2014-01-01), pages 661-668, XP055407314, JP ISSN: 0918-2918, DOI: 10.2169/internalmedicine.53.1293
- JOACHIM SEEGERS ET AL: "Natriuretic peptides for the detection of paroxysmal atrial fibrillation", OPEN HEART, vol. 2, no. 1, 1 August 2015 (2015-08-01), pages e000182-141, XP055407315, DOI: 10.1136/openhrt-2014-000182
- Serban Puricel ET AL: "A new tool to help diagnose patients suffering from paroxysmal palpitations? A handheld ECG device", , 1 January 2016 (2016-01-01), pages 296-298, XP055407316, Retrieved from the Internet: URL:https://cardiovascmed.ch/en/resource/j f/journal/file/download/article/cvm.2016.0 0444/cvm-00444.pdf/ [retrieved on 2017-09-18]
- JÜRGEN R. SCHAEFER ET AL: "Improved Detection of Paroxysmal Atrial Fibrillation Utilizing a Software-Assisted Electrocardiogram Approach", PLOS ONE, vol. 9, no. 2, 28 February 2014 (2014-02-28), page e89328, XP055278473, DOI: 10.1371/journal.pone.0089328
- EFTIHIA SBAROUNI ET AL: "Heart-type fatty acid binding protein in elective cardioversion of atrial fibrillation", CLINICAL BIOCHEMISTRY, ELSEVIER INC, US, CA, vol. 44, no. 12, 28 May 2011 (2011-05-28), pages 947-949, XP028239266, ISSN: 0009-9120, DOI: 10.1016/J.CLINBIOCHEM.2011.05.029 [retrieved on 2011-06-06]

## Description

The present invention relates to a method for diagnosing paroxysmal atrial fibrillation in a subject, comprising: determining the amount of FABP-3 (Fatty acid binding protein 3) in a sample from a subject suspected to suffer from paroxysmal atrial fibrillation, and comparing the determined amount to a reference amount. The method of the present invention may further comprise the determination of a BNP-type peptide.

Paroxysmal atrial fibrillation is defined as recurrent episodes of atrial fibrillation (AF) that terminate spontaneously in less than seven days, usually less than 24 hours. It may be either self-terminating or intermittent. Paroxysmal AF is common (5-10% of elderly subjects (see Prevalence, incidence and lifetime risk of atrial fibrillation: the Rotterdam study. Heeringa et al., Eur Heart J. 2006; 27(8):949)). Untreated patients have an increased stroke risk without the administration of anticoagulation therapy. The diagnosis of heart arrhythmia such as atrial fibrillation typically involves determination of the cause of the arrhythmia, and classification of the arrhythmia. The Gold Standard to detect AF is the electrocardiogram (ECG), preferably performed as 24 hour ECG (Holter Monitoring). However, Holter Monitoring can only detect AF if the arrhythmia occurs in the 24 hour period of ECG recording. Thus, paroxysmal AF is frequently not detected by Holter monitoring.

Various scientific publications deal with the association of biomarkers at enhanced levels with atrial fibrillation (reviewed in: Biomarkers in Atrial Fibrillation: Investigating Biologic Plausibility, Cause, and Effect R. Becker Journal of Thrombosis and Thrombolysis 19(1), 71-75, 2005).

The determination of NT-proBNP allows for the assessment of systolic dysfunction. Further, high sensitive Troponin assays allow the detection of subclinical cardiac injury. It has been described, that elevated levels of both, Troponin T and NT-proBNP independently predict a higher risk of a first recurrence of AF after 6 or 12 months (Circulating cardiovascular biomarkers in recurrent atrial fibrillation: data from the GISSI-Atrial Fibrillation Trial, R. Latini et al., JIntemMed2011;269:160-171). It has been further described, that patients may be selected based on cardiac protein levels, Troponin T and NT-proBNP to diagnose paroxysmal AF which has occurred before and is dating back even as long as about 1 week and to select patients who may benefit from anticoagulation therapy (WO 2014/072500).

The biomarker FABP-3 (Fatty acid binding protein 3), also known as Heart-type fatty acid binding protein (H-FABP), has been suggested as an early marker of myocardial infarction. FABP-3 is a low molecular weight cytoplasmic protein - human FABP-3 is a polypeptide of 132 amino acids and 14.8 KDa - and present abundantly in the myocardium. When the myocardium is injured, as in the case of myocardial infarction, low molecular weight cytoplasmic proteins including FABP-3 are released into the circulation and an elevated FABP-3 level is detectable in a blood sample (see e.g. Ruzgar et al., Heart Vessels, 21;209- 314 (2006). Other names for FABP-3 and H-FABP are: FABP-11 ( fatty acid binding protein 11), M-FABP (muscle fatty acid-binding protein), MDGI (mammary-derived growth inhibitor), and O-FABP.

Elevated circulating FABP-3 levels were observed in patients with ongoing Atrial Fibrillation (AF) compared with patients in sinus rhythm (Otaki et al., Internal medicine, (2014) Vol. 53, No. 7, pp. 661-8).

Elevated circulating levels of FABP-3 were observed to be associated to postoperative events of AF (Sezai et al., Carperitide and Atrial Fibrillation after Coronary Bypass Grafting: The Nihon University Working Group Study of Low-Dose HANP Infusion Therapy during Cardiac Surgery Trial for Postoperative Atrial Fibrillation. Circulation: Arrhythmia and Electrophysiology, (4 Jun 2015) Vol. 8, No. 3, pp. 546-553).

An increased level of FABP-3 was associated with AF after cardiac surgery, suggesting that ischemic myocardial damage is a contributing underlying mechanism (Rader et al., Perioperative heart-type fatty acid binding protein levels in atrial fibrillation after cardiac surgery. Heart rhythm: the official journal of the Heart Rhythm Society, (2013) Vol. 10, No. 2, pp.153-7).

Yang et al describe the combined use of a beta-blocker and the anti-ischemic drug trimetadizine. The 2016 guidelines indicate that trimetazidine may be considered for the treatment of stable angina pectoris with symptomatic HFrEF, when angina persists despite treatment with a beta-blocker (or alternative), to relieve angina (effective anti-anginal treatment, safe in HF). This recommendation is based on the body of evidence suggesting that trimetazidine may improve NYHA functional capacity, exercise duration and LV function in patients with HFrEF. There is no recommendation for trimetazidine in the setting of HF alone. (Yang et al. Efficacy of metoprolol in combination with trimetazidine in patients with atrial fibrillation and its effects on serum concentrations of H-FABP. South China Journal of Cardiovascular Diseases, (2014) 20(2), 168-170). The authors of the publication describe the use of trimetadizine in combination with a beta-blocker in patients with AF with the endpoints improvement of clinical symptoms, reduced recurrence of AF and reduced FABP3 levels. There is no hint, that FABP3 significantly associates with paroxysmal AF. Several different clinical parameters that are improved by these drugs may cause the reduction of FABP3 as well. Trimetazidine is an anti-ischemic drug, which improves myocardial glucose utilization through inhibition of fatty acid metabolism. FABP3 plays a role in the cytosolic transport of long-chain fatty acids and the protection of cardiac myocytes from fatty acids during ischemia. FABP3 as parameter of ischemic heart injury is suggested to correlate to anti-ischemic drug mechanism. Beta blockers are antihypertensive drugs. Blockade of beta-receptors reduces cardiac output and blood pressure as well as renal blood flow and the glomerular filtration rate of the kidney. Elevated FABP3 levels can result from reduced clearance due to kidney dysfunction. FABP3 as parameter of reduced glomerular filtration rate is suggested to correlate to antihypertensive drug mechanism.

US 2006/099608 and US 2007/218498 disclose FABP-3 within a list of several biomarkers for the diagnosis of vascular conditions.

US 2011/144205 discloses the screening of asymptomatic patients at risk of heart failure. FABP-3 is described within a list of several biomarkers.

US 2015/126385 discloses that FABP-3 may aid the diagnosis of ischemic stroke.

According to US2016/258965, FABP-3 may be indicative of the time that has elapsed since the onset of stroke, as one of several cardiac disorders.

Elevated circulating levels of FABP-3 were observed to be associated to the risk of recurrent stroke in patients with a history of a previous transient ischemic attack (Segal et al. Population-based study of blood biomarkers in prediction of subacute recurrent stroke. Stroke (2014) Vol. 45, No. 10, pp. 2912-2917).

A progressive increase of circulating levels among the subgroups of patients from paroxysmal to persistent or permanent atrial fibrillation is observed for most biomarkers including CRP (Li et al., Plasma oxidative stress and inflammatory biomarkers are associated with the sizes of the left atrium and pulmonary vein in atrial fibrillation patients. Clin Cardiol (2017) 40(2), pp. 89-942017, or Kossaify et al., Perspectives of biomarkers in acute cardiac care. Biomarker Insights (2013) 8, pp.115-126).

Thus, a significant increase of most AF biomarkers such as CRP is observed at later stages of atrial fibrillation.

However, an early diagnosis of atrial fibrillation, and thus the diagnosis of paroxysmal atrial fibrillation, is highly desired because atrial fibrillation is an important risk factor for stroke and systemic embolism. The most feared consequence of atrial fibrillation is (ischemic) stroke, which occurs when blood flow to the brain is blocked by a clot or by fatty deposits called plaque in the blood vessel lining. Regardless of symptomatology, the individuals with atrial fibrillation have a 5-fold increased risk of ischemic stroke. Moreover, strokes caused by complications from AF tend to be more severe than strokes with other underlying causes. Stroke caused by atrial fibrillation gives rise to more debilitating disabilities and has a higher mortality rate. At least 30% of all stroke patients are estimated to have atrial fibrillation, so that oral anticoagulation (OAC) for stroke prevention has become a beneficial, common treatment.

As can be derived from the above, the detection of paroxysmal atrial fibrillation is of particular relevance, but challenging. Accordingly, there is a need for reliable methods for the diagnosis of paroxysmal atrial fibrillation.

The technical problem underlying the present invention can be seen as the provision of methods for complying with the aforementioned need. The technical problem is solved by the embodiments characterized in the claims and herein below. The scope of the invention is defined by the appended claims. Embodiments and descriptions no longer falling under the scope of the appended claims are considered merely as examples suitable for understanding the invention.

Advantageously, it was found in the context of the studies of the present invention that the determination of the amount of FABP-3, in a sample from a subject allows for the diagnosis of paroxysmal atrial fibrillation. In contrast to several other biomarkers of AF, FABP-3 is already significantly increased in early stages of AF. Thanks to the present invention, it can be thus assessed whether a subject suffers from paroxysmal atrial fibrillation or not.

Accordingly, the present invention relates to a method for diagnosing paroxysmal atrial fibrillation in a subject, comprising:
(a) determining the amount of FABP-3 (Fatty acid binding protein 3) in a sample from a subject suspected to suffer from paroxysmal atrial fibrillation, and
(b) comparing the amount as determined in step a) to a reference amount.

In an embodiment of the method of the present invention, the method further comprises the determination of the amount of a BNP-type peptide in a sample from the subject in step a) and the comparison of the amount of said BNP-type peptide to a reference amount in step b).

Accordingly, the present invention relates to a method for diagnosing paroxysmal atrial fibrillation in a subject, comprising:
(a) determining the amount of FABP-3 (Fatty acid binding protein 3) and the amount of a BNP-type peptide in a sample from a subject suspected to suffer from paroxysmal atrial fibrillation, and
(b) comparing the amounts as determined in step a) to reference amounts.

In a preferred embodiment, the diagnosis of atrial fibrillation (AF) is based on the results of the comparison step b).

Accordingly, the present invention preferably comprises the steps of
a) determining the amount of FABP-3, and optionally the amount of a BNP-type peptide, in a sample from the subject,
b) comparing the amount of FABP-3 to a reference amount (for said marker) and optionally comparing the amount of the BNP-type peptide to a reference amount (for said marker), and
c) diagnosing atrial fibrillation based on the result(s) of the comparison step b).

The method as referred to in accordance with the present invention includes a method which essentially consists of the aforementioned steps or a method which includes further steps. Moreover, the method of the present invention, preferably, is an ex vivo and more preferably an in vitro method. Moreover, it may comprise steps in addition to those explicitly mentioned above. For example, the diagnosis of AF may be further based on the assessment of intermittent ECG recordings obtained from said subject over a period of at least one week by using a handheld ECG device (as described elsewhere herein in detail). Moreover, further steps may relate to the determination of further marker and/or to sample pre-treatments or evaluation of the results obtained by the method. The method may be carried out manually or assisted by automation. Preferably, step (a), (b) and/or (c) may in total or in part be assisted by automation, e.g., by a suitable robotic and sensory equipment for the determination in step (a) or a computer-implemented calculation in step (b).

Preferably, the term "diagnosing" as used herein, preferably, means assessing whether a subject as referred to in accordance with the method of the present invention suffers from paroxysmal atrial fibrillation (AF), or not. For example, the term refers to the assessment of the probability of the subject to suffer from paroxysmal AF, or not. Thus, it is assessed whether a subject has a low or a high probability of suffering from AF. A subject who is diagnosed to suffer from paroxysmal AF shall have a high probability of suffering from paroxysmal AF (such as a probability of about 80% or more). A subject who is diagnosed not to suffer from paroxysmal AF shall have a low probability of suffering from paroxysmal AF (such as a probability of about 15% or less than 15% ). Accordingly, the expression "diagnosing paroxysmal atrial fibrillation" as used herein shall be understood as "aiding" or "assisting" in the diagnosis of paroxysmal atrial fibrillation. E.g. a physician may be assisted in the diagnosis of paroxysmal atrial fibrillation.

As will be understood by those skilled in the art, the diagnosis of the present invention is usually not intended to be correct for 100% of the subjects to be tested. The term "diagnosing", preferably, requires that a correct diagnosis can be made for a statistically significant portion of subjects. Whether a portion is statistically significant can be determined without further ado by the person skilled in the art using various well known statistic evaluation tools, e.g., determination of confidence intervals, p-value determination, Student's t-test, Mann-Whitney test etc. Details are found in Dowdy and Wearden, Statistics for Research, John Wiley & Sons, New York 1983. Preferred confidence intervals are at least 90%, at least 95%, at least 97%, at least 98%, or at least 99%. The p-values are, preferably, 0.4, 0.1, 0.05, 0.01, 0.005, or 0.0001.

It is known in the art that biomarkers could be increased in various diseases and disorders. This does also apply to FABP-3 and BNP-type peptides. For example, FABP-3 is known to be increased in patients with acute coronary syndrome, whereas BNP-type peptides are known to be increased in patients with heart failure. However, this is taken into account by the skilled person.

The term "atrial fibrillation" ("abbreviated" AF or AFib) is well known in the art. As used herein, the term preferably refers to a supraventricular tachyarrhythmia characterized by uncoordinated atrial activation with consequent deterioration of atrial mechanical function. In particular, the term refers to an abnormal heart rhythm characterized by rapid and irregular beating. It involves the two upper chambers of the heart. In a normal heart rhythm, the impulse generated by the sino-atrial node spreads through the heart and causes contraction of the heart muscle and pumping of blood. In atrial fibrillation, the regular electrical impulses of the sino-atrial node are replaced by disorganized, rapid electrical impulses which result in irregular heart beats. Symptoms of atrial fibrillation are heart palpitations, fainting, shortness of breath, or chest pain. However, most episodes have no symptoms. On the electrocardiogram Atrial Fibrillation is characterized by the replacement of consistent P waves by rapid oscillations or fibrillatory waves that vary in amplitude, shape, and timing, associated with an irregular, frequently rapid ventricular response when atrioventricular conduction is intact.

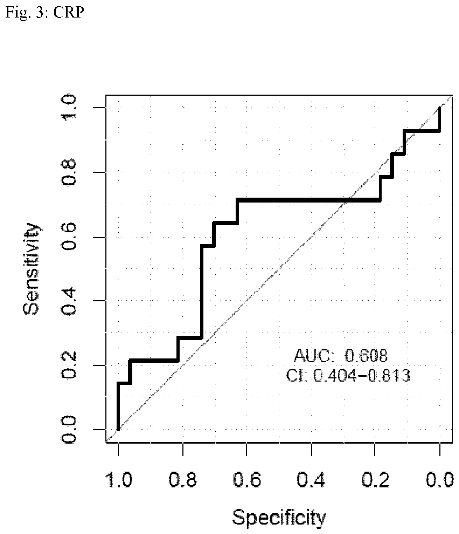

The American College of Cardiology (ACC), American Heart Association (AHA), and the European Society of Cardiology (ESC) propose the following classification system (see Fuster (2006) Circulation 114 (7): e257-354 see e.g. Figure 3 in the document): First detected AF, paroxysmal AF, persistent AF, and permanent AF.

All people with AF are initially in the category called first detected AF. However, the subject may or may not have had previous undetected episodes. A subject suffers from permanent AF, if the AF has persisted for more than one year. In particular, conversion back to sinus rhythm does not occur (or only with medical intervention). A subject suffers from persistent AF, if the AF lasts more than 7 days. The subject may require either pharmacologic or electrical intervention to terminate Atrial Fibrillation. Thus persistent AF occurs in episodes, but the arrhythmia does not convert back to sinus rhythm spontaneously. Paroxysmal Atrial Fibrillation, preferably, refers to an intermittent episode of Atrial Fibrillation which lasts up to 7 days. In most cases of paroxysmal AF, the episodes last less than 24 hours. The episodes of paroxysmal Atrial Fibrillation terminate spontaneously, i.e. without medical intervention. Paroxysmal AF is asymptomatic and underdiagnosed (silent AF). A preferred episode length of the present invention is shorter than 48 hrs, 24 hrs or 12 hrs (paroxysmal AF). Accordingly, the term "paroxysmal atrial fibrillation" as used herein is defined as episodes of AF that self-terminate, preferably, in less than 48 hours, more preferably in less than 24 hours, and, most preferably in less than 12 hours. Preferably, said episodes are recurrent. Further, it is envisaged that the episodes self-terminate in less than 6 hours.

Both persistent and paroxysmal AF may be recurrent within weeks or months, whereby distinction of paroxysmal and persistent AF is provided by ECG recordings: When a patient has had two or more episodes, AF is considered recurrent. If the arrhythmia terminates spontaneously, AF, in particular recurrent AF, is designated paroxysmal. AF is designated persistent if it lasts more than 7 days.

Whether a subject suffers from paroxysmal or persistent Atrial, or whether a subject does not suffer from AF can be confirmed by Epicardial High Density Mapping. This method which was applied in the Examples section is a different means for discrimination among subgroups of sinus rhythm, paroxysmal and persistent AF according to different conduction patterns of fibrillation waves resulting from electric activations (see Eckstein et al., Transmural Conduction Is the Predominant Mechanism of Breakthrough During Atrial Fibrillation Evidence From Simultaneous Endo-Epicardial High-Density Activation Mapping. Circ Arrhythm Electrophysiol. (2013) 6, pp. 334-341; van Marion et al., Diagnosis and Therapy of Atrial Fibrillation: the Past, the Present and the Future Diagnosis and Therapy of Atrial Fibrillation: the Past, the Present and the Future JAFIB: Journal of Atrial Fibrillation;Aug/Sep2015, Vol. 8 Issue 2, p5).

The term "subject" as referred to herein is, preferably, a mammal. Mammals include, but are not limited to, domesticated animals (e.g., cows, sheep, c ats, dogs, and horses), primates (e.g., humans and non-human primates such as monkeys), rabbits, and rodents (e.g., mice and rats). In a preferred embodiment, the subject is a human. The subject can be male or female.

In embodiment, the subject may be have at least one risk factor for paroxysmal atrial fibrillation. Preferred risk factor are age (see next paragraph, e.g. the subject is 65 years or older), hypertension, such as hypertension requiring anti-hypertensive medication, heart failure, such as heart failure AHA stage A - C, history of stroke, history of cardiac surgery or ablation. Preferably the subject may have also hints from remote electrocardiogram diagnostics, handheld devices, including Health Apps.

Preferably, the subject to be tested is 50 years of age or older, more preferably 60 years of age or older, and most preferably 65 years of age or older. Further, it is envisaged that the subject to be tested is 70 years of age or older.

As set forth above, the biomarker FABP-3 could be increased in various diseases and disorders other than atrial fibrillation. In an embodiment of the present invention, it is envisaged that the subject does not suffer from such diseases and disorders. For example, it is envisaged that the subject shall not suffer form an acute coronary syndrome.

In an embodiment of the method of the present invention, the subject to be tested suffers from cardiac disease, in particular from cardiac disease requiring valve surgery, or cardiac disease requiring coronary artery bypass surgery. Thus, the subject may suffer from coronary artery disease, i.e. stable coronary artery disease. The subject may have various degrees of cardiovascular risk profile up to patients with severe heart failure. The subject may have no cardiovascular disease.

Preferably, a subject who is suspected to suffer from paroxysmal atrial fibrillation is a subject who shows at least one symptom of atrial fibrillation and/or who has shown at least one symptom of atrial fibrillation prior to carrying out the method for assessing paroxysmal atrial fibrillation. Said symptoms are usually transient and may arise in a few seconds and may disappear just as quickly. Symptoms of atrial fibrillation include dizziness, fainting, shortness of breath and, in particular, heart palpitations. Accordingly, the at least one symptom of atrial fibrillation is selected from dizziness, fainting, shortness of breath and, in particular, heart palpitations. Preferably, the subject has shown at least one symptom of atrial fibrillation within six months, more preferably within one month, even more preferably within two weeks, and most preferably within one week prior to obtaining the sample. In particular, it is envisaged that the subject has shown at least one symptom of AF within 2 days prior to obtaining the sample. Also, it is envisaged that the subject has shown at least one symptom of AF within 24 hours, or even within 12 hours prior to obtaining the sample. Thus, the subject is suspected to have exhibited an episode of atrial fibrillation, i.e. paroxysmal atrial fibrillation, within one of these periods. Accordingly, the steps carried out in accordance with the aforementioned method of the present invention allow for the diagnosis (or to be more precise for an aid in the diagnosis) whether the subject recently has suffered from an episode of atrial fibrillation, or not, within these periods, in particular, whether the subject has suffered from an episode of atrial fibrillation within one week, in particular within two days or within 24 hours or within 12 hours prior to obtaining the sample. Thus, the present invention also contemplates a method for diagnosing a recent episode of atrial fibrillation, in particular of paroxysmal atrial fibrillation.

In a preferred embodiment of the method of the present invention, the subject has no known history of atrial fibrillation, in particular paroxysmal atrial fibrillation. Accordingly, the subject shall not have been diagnosed to suffer from atrial fibrillation previously, i.e. before carrying out the method of the present invention (in particular before obtaining the sample from the subject). However, the subject may or may not have had previous undiagnosed episodes of atrial fibrillation.

In an embodiment of the method or use of the present invention, the subject has conduction disturbances. Such conduction disturbances could be confirmed by invasive electrical mapping of atrial fibrillation during a cardiac surgery (see Examples section). Fatty infiltration is one important determinant of conduction disturbances as electrical barrier impeding impulse propagation.

Further, the subject to be tested, preferably, does not suffer from permanent atrial fibrillation and from persistent atrial fibrillation. Thus, the subject suffers neither from permanent AF nor from persistent AF. Further, it is envisaged that the subject does not suffer from atrial flutter.

In particular, it is envisaged that the subject has not been subjected to cardioversion prior to carrying out carrying the method of the present invention (in particular before the sample to be tested has been obtained). Cardioversion is a medical procedure by which cardiac arrhythmia is converted to a normal rhythm. This can be achieved by electrical cardioversion or cardioversion with an antiarrhythmic agent.

Preferably, the patient is in sinus rhythm at the time at which the sample is obtained (i.e. in normal sinus rhythm). Accordingly, the subject preferably does not suffer from an episode of atrial fibrillation at the time at which the sample is obtained.

Criteria for normal sinus rhythm include Normal heart rate (classically 60 to 100 beats per minute for an adult), Regular rhythm, with less than 0.16 second variation in the shortest and longest durations between successive P waves, the sinus node should pace the heart - therefore, P waves must be round, all the same shape, and present before every QRS complex in a ratio of 1:1, Normal P wave axis (0 to +75 degrees). Normal PR interval, QRS complex and QT interval, QRS complex positive in leads I, II, aVF and V3-V6, and negative in lead aVR.

The term "sample" refers to a sample of a body fluid.

Samples of body fluids can be obtained by well-known techniques and include samples of blood, plasma and serum.

The sample is a blood (i.e. whole blood), serum or plasma sample. Serum is the liquid fraction of whole blood that is obtained after the blood is allowed to clot. For obtaining the serum, the clot is removed by centrifugation and the supernatant is collected. Plasma is the acellular fluid portion of blood. For obtaining a plasma sample, whole blood is collected in anticoagulant-treated tubes (e.g. citrate-treated or EDTA-treated tubes). Cells are removed from the sample by centrifugation and the supernatant (i.e. the plasma sample) is obtained.

In a preferred embodiment of the method of the present invention, the method further comprises in step a) the determination of the amount of a BNP-type peptide in a sample from the subject and in step b) the comparison of the amount of the of the BNP-type peptide to a reference amount, i.e. to a reference amount for said BNP-type peptide.

The biomarkers that are determined in connection with the method of the present invention are well known in the art:
The term "FABP-3" as used herein refers to the fatty acid binding protein 3. FABP-3 is also known as heart fatty acid binding protein or heart type fatty acid binding protein (abbreviated H-FABP). Preferably, the term also includes variants of FABP-3. FABP-3 as used herein, preferably, relates to human FABP-3. The DNA sequence of the polypeptide encoding the human FABP-3 polypeptide as well the protein sequence of human FABP-3 is well known in the art and was first described by Peeters et al. (Biochem. J. 276 (Pt 1), 203-207 (1991)). Moreover, the sequence of human H-FABP can be found, preferably, in Genbank entry U57623.1 (cDNA sequence) and AAB02555.1 (protein sequence). The major physiological function of FABP is thought to be the transport of free fatty acids, see e.g. Storch et al., Biochem. Biophys. Acta. 1486 (2000), 28-44. Other names for FABP-3 and H-FABP are: FABP-11 (fatty acid binding protein 11), M-FABP (muscle fatty acid-binding protein), MDGI (mammary-derived growth inhibitor), and O-FABP.

In a preferred embodiment of the present invention, the amount of the FABP-3 polypeptide is determined. In another embodiment of the present invention, the amount of the FABP-3 transcript is determined.

The Brain Natriuretic Peptide type peptide (herein also referred to as BNP-type peptide) is preferably selected from the group consisting of pre-proBNP, proBNP, NT-proBNP, and BNP. The pre-pro peptide (134 amino acids in the case of pre-proBNP) comprises a short signal peptide, which is enzymatically cleaved off to release the pro peptide (108 amino acids in the case of proBNP). The pro peptide is further cleaved into an N-terminal pro peptide (NT-pro peptide, 76 amino acids in case of NT-proBNP) and the active hormone (32 amino acids in the case of BNP). Preferably, brain natriuretic peptides according to the present invention are NT-proBNP, BNP, and variants thereof. BNP is the active hormone and has a shorter half-life than its respective inactive counterpart NT-proBNP. Preferably, the Brain Natriuretic Peptide-type peptide is BNP (Brain natriuretic peptide), and more preferably NT-proBNP (N-terminal of the prohormone brain natriuretic peptide).

The term "determining" the amount of a biomarker as referred to herein, i.e. of FABP-3 or a BNP-type peptide, refers to the quantification of the biomarker, e.g. to determining the level of the biomarker in the sample, employing appropriate methods of detection described elsewhere herein. The terms "measuring" and "determining" are used herein interchangeably (in connection with biomarkers).

In an embodiment, the amount of a biomarker is determined by contacting the sample with an agent that specifically binds to the biomarker, thereby forming a complex between the agent and said biomarker, detecting the amount of complex formed, and thereby measuring the amount of said biomarker.

The FABP-3 polypeptide or the BNP-type peptide can be detected using methods generally known in the art. Methods of detection generally encompass methods to quantify the amount of a biomarker in the sample (quantitative method). It is generally known to the skilled artisan which of the following methods are suitable for qualitative and/or for quantitative detection of a biomarker. Samples can be conveniently assayed for, e.g., proteins using Westerns and immunoassays, like ELISAs, RIAs, fluorescence- and luminescence-based immunoassays, which are commercially available. Further suitable methods to detect a biomarker include measuring a physical or chemical property specific for the peptide or polypeptide such as its precise molecular mass or NMR spectrum. Said methods comprise, e.g., biosensors, optical devices coupled to immunoassays, biochips, analytical devices such as mass-spectrometers, NMR- analyzers, or chromatography devices. Further, methods include microplate ELISA-based methods, fully-automated or robotic immunoassays (available for example on Elecsys^{™} analyzers), CBA (an enzymatic Cobalt Binding Assay, available for example on Roche-Hitachi^{™} analyzers), and latex agglutination assays (available for example on Roche-Hitachi^{™} analyzers).

For the detection of biomarker proteins as referred to herein a wide range of immunoassay techniques using such an assay format are available, see, e.g., U.S. Pat. Nos. 4,016,043, 4,424,279, and 4,018,653. These include both single-site and two-site or "sandwich" assays of the non-competitive types, as well as in the traditional competitive binding assays. These assays also include direct binding of a labeled antibody to a target biomarker.

Sandwich assays are among the most useful immunoassays.

Methods employing electrochemiluminescent labels are well-known. Such methods make use of the ability of special metal complexes to achieve, by means of oxidation, an excited state from which they decay to ground state, emitting electrochemiluminescence. For review see Richter, M.M., Chem. Rev. 104 (2004) 3003-3036.

In an embodiment, the detection antibody (or an antigen-binding fragment thereof) to be used for measuring the amount of a biomarker is ruthenylated or iridinylated. Accordingly, the antibody (or an antigen-binding fragment thereof) shall comprise a ruthenium label. In an embodiment, said ruthenium label is a bipyridine-ruthenium(II) complex. Or the antibody (or an antigen-binding fragment thereof) shall comprise an iridium label. In an embodiment, said iridium label is a complex as disclosed in WO 2012/107419.

Measuring the amount of a polypeptide (such as FABP-3 or the BNP-type peptide) may, preferably, comprise the steps of (a) contacting the polypeptide with an agent that specifically binds said polypeptide, (b) (optionally) removing non-bound agent, (c) measuring the amount of bound binding agent, i.e. the complex of the agent formed in step (a). According to a preferred embodiment, said steps of contacting, removing and measuring may be performed by an analyzer unit. According to some embodiments, said steps may be performed by a single analyzer unit of said system or by more than one analyzer unit in operable communication with each other. For example, according to a specific embodiment, said system disclosed herein may include a first analyzer unit for performing said steps of contacting and removing and a second analyzer unit, operably connected to said first analyzer unit by a transport unit (for example, a robotic arm), which performs said step of measuring.

The agent which specifically binds the biomarker (herein also referred to as "binding agent") may be coupled covalently or non-covalently to a label allowing detection and measurement of the bound agent. Labeling may be done by direct or indirect methods. Direct labeling involves coupling of the label directly (covalently or non-covalently) to the binding agent. Indirect labeling involves binding (covalently or non-covalently) of a secondary binding agent to the first binding agent. The secondary binding agent should specifically bind to the first binding agent. Said secondary binding agent may be coupled with a suitable label and/or be the target (receptor) of tertiary binding agent binding to the secondary binding agent. Suitable secondary and higher order binding agents may include antibodies, secondary antibodies, and the well-known streptavidin-biotin system (Vector Laboratories, Inc.). The binding agent or substrate may also be "tagged" with one or more tags as known in the art. Such tags may then be targets for higher order binding agents. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium complexes, iridium complexes, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star^{™} (Amersham Biosciences), ECF^{™} (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enzymatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. A radioactive label can be detected by any method known and appropriate, e.g. a light-sensitive film or a phosphor imager.

The amount of a polypeptide may be, also preferably, measured as follows: (a) contacting a solid support comprising a binding agent for the polypeptide as described elsewhere herein with a sample comprising the peptide or polypeptide and (b) measuring the amount of peptide or polypeptide which is bound to the support. Materials for manufacturing supports are well-known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc.

In yet an aspect the sample is removed from the complex formed between the binding agent and the at least one marker prior to the measurement of the amount of formed complex. Accordingly, in an aspect, the binding agent may be immobilized on a solid support. In yet an aspect, the sample can be removed from the formed complex on the solid support by applying a washing solution.

"Sandwich assays" are among the most useful and commonly used assays encompassing a number of variations of the sandwich assay technique. Briefly, in a typical assay, an unlabeled (capture) binding agent is immobilized or can be immobilized on a solid substrate, and the sample to be tested is brought into contact with the capture binding agent. After a suitable period of incubation, for a period of time sufficient to allow formation of a binding agent-biomarker complex, a second (detection) binding agent labeled with a reporter molecule capable of producing a detectable signal is then added and incubated, allowing time sufficient for the formation of another complex of binding agent-biomarker-labeled binding agent. Any unreacted material may be washed away, and the presence of the biomarker is determined by observation of a signal produced by the reporter molecule bound to the detection binding agent. The results may either be qualitative, by simple observation of a visible signal, or may be quantitated by comparison with a control sample containing known amounts of biomarker.

The incubation steps of a typical sandwich assay can be varied as required and appropriate. Such variations include for example simultaneous incubations, in which two or more of binding agent and biomarker are co-incubated. For example, both, the sample to be analyzed and a labeled binding agent are added simultaneously to an immobilized capture binding agent. It is also possible to first incubate the sample to be analyzed and a labeled binding agent and thereafter add an antibody bound to a solid phase or capable of binding to a solid phase.

The formed complex between a specific binding agent and the biomarker shall be proportional to the amount of the biomarker present in the sample. It will be understood that the specificity and/or sensitivity of the binding agent to be applied defines the degree of proportion of at least one marker comprised in the sample which is capable of being specifically bound. Further details on how the measurement can be carried out are also found elsewhere herein. The amount of formed complex shall be transformed into an amount of the biomarker reflecting the amount indeed present in the sample.

The terms "binding agent", "specific binding agent", "analyte-specific binding agent", "detection agent" and "agent that specifically binds to a biomarker" are used interchangeably herein. Preferably it relates to an agent that comprises a binding moiety which specifically binds the corresponding biomarker. Examples of "binding agents" or "agents" are a nucleic acid probe, nucleic acid primer, DNA molecule, RNA molecule, aptamer, antibody, antibody fragment, peptide, peptide nucleic acid (PNA) or chemical compound. A preferred agent is an antibody which specifically binds to the biomarker to be measured. The term "antibody" herein is used in the broadest sense and encompasses various antibody structures, including but not limited to monoclonal antibodies, polyclonal antibodies, multi-specific antibodies (e.g., bispecific antibodies), and antibody fragments so long as they exhibit the desired antigen-binding activity (i.e. antigen-binding fragments thereof). Preferably, the antibody is a polyclonal antibody. More preferably, the antibody is a monoclonal antibody.

The term "specific binding" or "specifically bind" refers to a binding reaction wherein binding pair molecules exhibit a binding to each other under conditions where they do not significantly bind to other molecules. The term "specific binding" or "specifically binds", when referring to a protein or peptide as biomarker, refers to a binding reaction wherein a binding agent binds to the corresponding biomarker with an affinity of at least 10⁻⁷ M. The term "specific binding" or "specifically binds" preferably refers to an affinity of at least 10⁻⁸ M or even more preferred of at least 10⁻⁹ M for its target molecule. The term "specific" or "specifically" is used to indicate that other molecules present in the sample do not significantly bind to the binding agent specific for the target molecule.

The term "amount" as used herein encompasses the absolute amount of the biomarker, the relative amount or concentration of the biomarker as well as any value or parameter which correlates thereto or can be derived therefrom. Such values or parameters comprise intensity signal values from all specific physical or chemical properties obtained from the said peptides by direct measurements, e.g., intensity values in mass spectra or NMR spectra. Moreover, encompassed are all values or parameters which are obtained by indirect measurements specified elsewhere in this description, e.g., response amounts measured from biological read out systems in response to the peptides or intensity signals obtained from specifically bound ligands. It is to be understood that values correlating to the aforementioned amounts or parameters can also be obtained by all standard mathematical operations.

The term "comparing" as used herein refers to comparing the amount of the FABP-3 polypeptide or the BNP-type peptide in the sample from the subject with the reference amount of the biomarker. It is to be understood that comparing as used herein usually refers to a comparison of corresponding parameters or values, e.g., an absolute amount is compared to an absolute reference amount while a concentration is compared to a reference concentration or an intensity signal obtained from the biomarker in a sample is compared to the same type of intensity signal obtained from a reference sample. The comparison may be carried out manually or computer-assisted. Thus, the comparison may be carried out by a computing device. The value of the measured or detected amount of the biomarker in the sample from the subject and the reference amount can be, e.g., compared to each other and the said comparison can be automatically carried out by a computer program executing an algorithm for the comparison. The computer program carrying out the said evaluation will provide the desired assessment in a suitable output format. For a computer-assisted comparison, the value of the measured amount may be compared to values corresponding to suitable references which are stored in a database by a computer program. The computer program may further evaluate the result of the comparison, i.e. automatically provide the desired assessment in a suitable output format. For a computer-assisted comparison, the value of the measured amount may be compared to values corresponding to suitable references which are stored in a database by a computer program. The computer program may further evaluate the result of the comparison, i.e. automatically provide the desired assessment in a suitable output format.

In accordance with the present invention the amount of FABP-3 and optionally the amount of BNP-type peptide (in particular the amount of the biomarker NT-proBNP) shall be compared to a reference. The reference is preferably a reference amount. The term "reference amount" as used herein refers to an amount which allows for allocation of a subject into either (i) the group of subjects suffering from paroxysmal atrial fibrillation or (ii) the group of subjects not suffering from paroxysmal atrial fibrillation. A suitable reference amount may be determined from a reference sample to be analyzed together, i.e. simultaneously or subsequently, with the test sample.

Reference amounts can, in principle, be calculated for a cohort of subjects as specified above based on the average or mean values for a given biomarker by applying standard methods of statistics. In particular, accuracy of a test such as a method aiming to diagnose an event, or not, is best described by its receiver-operating characteristics (ROC) (see especially Zweig 1993, Clin. Chem. 39:561-577). The ROC graph is a plot of all of the sensitivity versus specificity pairs resulting from continuously varying the decision threshold over the entire range of data observed. The clinical performance of a diagnostic method depends on its accuracy, i.e. its ability to correctly allocate subjects to a certain prognosis or diagnosis. The ROC plot indicates the overlap between the two distributions by plotting the sensitivity versus 1 - specificity for the complete range of thresholds suitable for making a distinction. On the y-axis is sensitivity, or the true-positive fraction, which is defined as the ratio of number of true-positive test results to the product of number of true-positive and number of false-negative test results. This has also been referred to as positivity in the presence of a disease or condition. It is calculated solely from the affected subgroup. On the x-axis is the false-positive fraction, or 1 - specificity, which is defined as the ratio of number of false-positive results to the product of number of true-negative and number of false-positive results. It is an index of specificity and is calculated entirely from the unaffected subgroup. Because the true- and false-positive fractions are calculated entirely separately, by using the test results from two different subgroups, the ROC plot is independent of the prevalence of the event in the cohort. Each point on the ROC plot represents a sensitivity/1 - specificity pair corresponding to a particular decision threshold. A test with perfect discrimination (no overlap in the two distributions of results) has a ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. If the ROC plot falls completely below the 45° diagonal, this is easily remedied by reversing the criterion for "positivity" from "greater than" to "less than" or vice versa. Qualitatively, the closer the plot is to the upper left corner, the higher the overall accuracy of the test. Dependent on a desired confidence interval, a threshold can be derived from the ROC curve allowing for the diagnosis for a given event with a proper balance of sensitivity and specificity, respectively. Accordingly, the reference to be used for the aforementioned method of the present invention, i.e. a threshold which allows to differentiate between subjects suffering from paroxysmal atrial fibrillation or those not suffering from paroxysmal atrial fibrillation among a cohort of subjects can be generated, preferably, by establishing a ROC for said cohort as described above and deriving a threshold amount therefrom. Dependent on a desired sensitivity and specificity for a diagnostic method, the ROC plot allows deriving a suitable threshold. It will be understood that an optimal sensitivity is desired for excluding a subject suffering from paroxysmal atrial fibrillation (i.e. a rule out), whereas an optimal specificity is envisaged for a subject to be assessed as suffering from paroxysmal atrial fibrillation (i.e. a rule in). In particular, it is envisaged that the subject is assessed as suffering from paroxysmal atrial fibrillation.

In preferred embodiments, the term "reference amount" herein refers to a predetermined value. In a preferred embodiment, said predetermined value shall allow for differentiating between a subject suffering from paroxysmal atrial fibrillation and a subject not suffering from paroxysmal atrial fibrillation (optionally in combination with the results of the ECG recordings). In another embodiment, said reference amount shall allow for diagnosing that a subject does not suffer from paroxysmal atrial fibrillation, i.e. for excluding a subject suffering from atrial fibrillation.

In certain embodiments, the reference amount is derived from a subject or a group of subjects known not to suffer from paroxysmal atrial fibrillation or from a subject or a group of subjects known to suffer from paroxysmal atrial fibrillation.

Preferably, an amount of FABP-3 in the sample from the subject which is larger than the reference amount indicates that the subject suffers from paroxysmal atrial fibrillation.

Also preferably, an amount of FABP-3 in the sample from the subject which is lower than the reference amount indicates that the subject does not suffer from paroxysmal atrial fibrillation.

The following preferably applies, if FABP-3 and a BNP-type peptide are determined:
Preferably, an amount of FABP-3 in the sample from the subject which is larger than the reference amount and an amount of BNP-type peptide in the sample from the subject which is larger than the reference amount indicates that the subject suffers from paroxysmal atrial fibrillation. Thus both amounts are increased.

Also preferably, an amount of FABP-3 in the sample from the subject which is lower than the reference amount and an amount of BNP-type peptide in the sample from the subject which is lower than the reference amount indicate that the subject does not suffer from paroxysmal atrial fibrillation.

In a preferred embodiment of the method of the present invention, the method further comprises the assessment of intermittent ECG recordings obtained from said subject over a period of at least one week by using a handheld ECG device. In this embodiment, the diagnosis whether the subject suffers from paroxysmal atrial fibrillation, or not, is based on the results of the comparison step (b), i.e. of the amount of FABP-3 to the reference amount, and optionally of the amount of the BNP-type peptide to the reference amount, and the assessment of intermittent ECG recordings.

Electrocardiography (abbreviated ECG) is the process of recording the electrical activity of the heart. An ECG device records the electrical signals produced by the heart which spread throughout the body to the skin. The recording is of the electrical signal is achieved by contacting the skin of the test subject with electrodes comprised by the ECG device. The process of obtaining the recording is non-invasive and risk-free.

In accordance with the present invention the recordings obtained with a handheld ECG device shall be assessed. The expression "assessing intermittent ECG recordings" as used herein preferably refers to assessing the presence or absence of atrial fibrillation in the intermittent ECG recordings (i.e. ECG readings/ECG strips) obtained from the subject. Thus, the recordings are interpreted. Said assessment can be carried out by a physician such as a cardiologist and/or can be computer-assisted. For example, a pre-assessment can be done by an automated software. The final assessment of the pre-assessed results can be carried out by a physician.

The handheld ECG device as referred to in the method of the present invention thus shall allow for the diagnosis of atrial fibrillation, i.e. for the assessment of the presence or absence of atrial fibrillation (in the ECG recordings obtained from the subject).

Handheld ECG devices which allow for the diagnosis of AF are produced by various manufacturers. Examples are the AfibAlert^{®} from Lohman Technologies a device specifically targeting the detection of atrial fibrillation, the Zenicor-ECG from Zenicor, Sweden, the AliveCor Mobile ECG, the Dimetek Micro Ambulatory ECG Recorder, the ECG Check, the HeartCheck^{™} PEN handheld ECG, the InstantCheck Real Time Display ECG monitor, the ReadMyHeart device, or the REKA E100.

In one embodiment of the method of the present invention, the handheld ECG device is a one-lead device. In another preferred embodiment, the ECG device used for obtaining the intermittent ECG recordings is a 12-lead ECG device.

In an embodiment of the present invention, the handheld ECG device is a device operated by the subject to be tested. Thus, the handheld device as referred to in the method of the present invention shall allow for self-testing by the test subject. In other words, the recordings to be assessed were obtained by self-testing.

In accordance with the present invention, it is envisaged that the handheld ECG device comprises two dry-contact electrodes. Thus, it is contemplated that the intermittent ECG recordings have been obtained by placing two fingers (such as the left and right thumb) on the dry-contact electrodes. Alternatively, the dry-contact electrodes can be placed against the bare skin such as the chest.

The ECG recordings in accordance with the present invention shall be intermittent ECG recordings. Thus, the ECG recordings shall not have been obtained continuously from the subject (which is e.g. the case for Holter monitoring). Accordingly, the ECG recordings to be assessed are recordings that were obtained periodically over a period of at least one week.

However, it is envisaged that ECG recordings were obtained at least once a day from the subject. The device is thus operated by the subject as least once a day. The expression "at least once" as used herein means once or more than once such as twice, three times, four times, five times etc. In an embodiment, the ECG recordings are obtained twice a day or more. The recordings may be stored or transmitted to an ECG database e.g. via mobile phone that is built in or connected with the ECG device. For example, the ECG device can be connected with a mobile phone (e.g. via a bluetooth or wireless connection) which transfers recordings to the database. The transmission can be achieved via an app (such as an Android or iOS app). After transmission of the recordings to the database, the recordings can be assessed for the presence or absence of atrial fibrillation, i.e. interpreted, by a physician.

The length of a (single) recording is usually between about 10 seconds and about 120 seconds. In an embodiment the length of a recording is between about 20 second and about 60 seconds. For example, an ECG recording may have been obtained by placing the thumbs on the two electrodes for about 30 seconds.

Further, it is in particular envisaged that the intermittent ECG recordings were obtained when the subject shows symptoms of atrial fibrillation (provided that the subject has shown symptoms). The symptoms are described elsewhere herein. Said symptoms are usually transient and may arise in a few seconds and may disappear just as quickly. If the subject does not show symptoms of atrial fibrillation, the recordings shall be nevertheless obtained at least once a day as described above.

In an embodiment, the intermittent, i.e. non-continuous, ECG recordings shall have been obtained over a period of at least four days. In another embodiment, the intermittent, i.e. non-continuous, ECG recordings shall have been obtained over a period of at least one week. Thus, there shall be at least seven, in particular at least 14 recordings that shall be assessed. In an embodiment, the intermittent ECG recordings shall have been obtained over a period of at least ten days. In another embodiment, the intermittent ECG recordings shall have been obtained over a period of at least two weeks. Further, it is envisaged that the intermittent ECG recordings to be assessed have been obtained over a period of one week to two weeks, in particular over a period of ten days to two weeks. The presence of AF in at least one recording is indicative for the diagnosis of AF, in particular paroxysmal AF.

If the method comprises the assessment of intermittent ECG recordings as set forth above, the diagnosis that the subject suffers from atrial fibrillation or not shall be based on the results of the comparison step b) and the assessment of the intermittent ECG recordings:
In an embodiment, it is diagnosed that the patient does not suffer from paroxysmal atrial fibrillation (ruling out paroxysmal atrial fibrillation). This is indicated by the absence of atrial fibrillation in the intermittent ECG recordings and by an amount of FABP-3 (and by an amount of the BNP-type peptide, if the BNP-type peptide is determined) in the sample from the subject lower than the reference amount. In other words, the absence of atrial fibrillation in the intermittent ECG recordings in combination with an amount of FABP-3 (and optionally the BNP-type peptide) below the reference are indicative for a subject who does not suffer from paroxysmal atrial fibrillation. Thus, the subject has a low probability of suffering from paroxysmal atrial fibrillation (such as a probability of lower than 10 or 15%).

In an embodiment, it is diagnosed that the patient suffers from paroxysmal atrial fibrillation. This is indicated by the presence of atrial fibrillation in the intermittent ECG recordings (i.e. the presence of atrial fibrillation in at least one recording) and/or by an amount of FABP-3 (and optionally by an amount of the BNP-type peptide) in the sample from the subject above the reference. In other words, the presence of atrial fibrillation in the intermittent ECG recordings and/or an amount of FABP-3 (and optionally an amount of the BNP-type peptide) above the reference are indicative for a subject who suffers from paroxysmal atrial fibrillation. Thus, the subject has a high probability of suffering from paroxysmal atrial fibrillation (such as a probability of higher than 70 or 80%).

The absence of atrial fibrillation in the intermittent ECG recordings from the subject in combination with an amount of FABP-3 above the reference might be indicative for a subject who suffers from atrial fibrillation. Although, no atrial fibrillation was detected in the ECG, the subject is likely to suffer from atrial fibrillation. This could be confirmed by continuously monitoring electrical activity of the cardiovascular system for at least 24 hours, e.g. by Holter monitoring or by implantable loop recorders (ILR).

In an embodiment of the method said method further comprises a step of recommending and/or initiating at least one suitable supportive measure based on the results of the diagnosis. Said at least one suitable supportive measure shall be recommended or initiated to subjects diagnosed to suffer from atrial fibrillation, i.e. to a subject who has a high probability of suffering from AF.

The term "recommending" as used herein means establishing a proposal for a suitable supportive measure which could be applied to the subject who has been diagnosed to suffer from paroxysmal AF.

The suitable supportive measure refers to all measures which can be applied to subjects suffering from AF, i.e. having a high probability of suffering from paroxysmal AF. For example, patient management measures include the verification of the diagnosis of paroxysmal AF (e.g. by Holter monitoring) and/or drug treatment.

If the subject suffers from paroxysmal atrial fibrillation, a therapy with at least one anticoagulant shall be recommended or initiated. The anticoagulant shall aim to reduce or prevent coagulation of blood and related stroke. Preferably, the anticoagulant is selected from the group consisting of heparin, a coumarin derivative, such as warfarin or dicumarol, tissue factor pathway inhibitor (TFPI), antithrombin III, factor IXa inhibitors, factor Xa inhibitors, inhibitors of factors Va, and thrombin inhibitors.

In a preferred embodiment, the anticoagulant is a Non-Vitamin K antagonist oral anticoagulants (abbreviated NOAC). Such anticoagulants inhibit clot formation and unlike warfarin are not Vitamin K antagonists. Such anticoagulants are well known in the art and described e.g. in Hijazi et al., 2015. The ABC (age, biomarkers, clinical history) stroke risk score: a biomarker-based risk score for predicting stroke in atrial fibrillation. Preferred NOACs are dabigatran, rivaroxaban, and apixaban.

Accordingly, the present disclosure further relates to a method of recommending and/or initiating at least one suitable supportive measure as set forth above. In an embodiment, the method comprises the steps of the method of the present invention, i.e. the method of diagnosing paroxysmal atrial fibrillation, as set forth herein elsewhere and the further step of recommending and/or initiating the at least one suitable supportive measure. In an embodiment, the paroxysmal atrial fibrillation is treated in the subject by administering an anticoagulant.

The present invention further concerns a method of aiding in the diagnosis of atrial fibrillation, said method comprising the steps of:
a) determining the amount of the biomarker FABP-3 and optionally the amount of a BNP-type peptide in a provided or obtained sample from a subject suspected to suffer from paroxysmal atrial fibrillation, and
b) providing information on the determined amount of the biomarker FABP-3 and optionally on the determined amount of the BNP-type peptide to the attending physician of the subject, thereby aiding in the diagnosis of paroxysmal atrial fibrillation in said subject.

The attending physician shall be the physician who requested the determination of the biomarker(s). The aforementioned method shall aid the attending physician in the diagnosis of paroxysmal atrial fibrillation.

Step a) of the aforementioned method of obtaining the sample does not encompass the drawing of the sample from the subject. Preferably, the sample is obtained by receiving a sample from said subject. Thus, the sample can have been delivered.

The present disclosure further relates to a method, comprising:
a) providing a test for the biomarker FABP-3 and optionally a test for the a BNP-type peptide. and
b) providing instructions for using of test results obtained or obtainable by said test(s) in the diagnosis of paroxysmal atrial fibrillation.

The purpose of the aforementioned method is, preferably, the aid in the diagnosis of paroxysmal atrial fibrillation.

The instructions shall contain a protocol for carrying out the method of diagnosing paroxysmal atrial fibrillation as described herein above. Further, the instructions shall contain at least one value for a reference amount for FABP-3 and optionally at least one value for a reference amount for a BNP-type peptide.

The "test" is preferably a kit adapted to carry out the method of diagnosing paroxysmal atrial fibrillation. Said kit shall comprise at least one detection agent for the biomarker FABP-3 and optionally at least one detection agent for a BNP-type peptide. The detection agents for the two biomarkers can be provided in a single kit or in two separate kits.

The test result obtained or obtainable by said test, is the value for the amount of the biomarker(s) in the sample from the subject.

Moreover, the present invention relates to the use of
i) the biomarker FABP-3 and optionally a BNP-type peptide and/or
ii) at least one detection agent that specifically binds to FABP-3, and optionally at least one detection agent that specifically binds to a BNP-type peptide,
in a sample from a subject for diagnosing paroxysmal atrial fibrillation (in said subject).

The aforementioned use may further encompass the use of handheld ECG device and/or intermittent ECG recordings obtained by using a handheld ECG device from the obtained from said subject over a period of at least one week by using a handheld ECG device.

The terms mentioned in connection with the aforementioned use such as "sample", "subject", "detection agent", "FABP-3", "specifically binding", "paroxysmal atrial fibrillation", and "have been defined in connection with the method for diagnosing paroxysmal atrial fibrillation. The definitions and explanations apply accordingly.

In the Figures:
Fig. 1: Diagnosis of paroxysmal AF with elevated levels of circulating FABP-3 (Median values: 1.92 ng/mL in samples of 30 patients not suffering from AF, 2.84 ng/mL in samples of 14 patients with paroxysmal AF; 2.75 ng/mL in samples of 16 patients with persistent AF).
Fig. 2: Results for NT-proBNP in plasma samples of the patients described in the Figure legend of Figure 1.
Fig. 3: Results for hsCRP (plasma samples) in plasma samples of the 14 patients suffering from paroxysmal AF and in plasma samples of the 30 patients not suffering from AF described in the legend of Figure 1.
Fig. 4: Differential expression of FABP-3 in tissue sample of the right atrial appendage of patients with AF (case) or in sinus rhythm (control) based on RNAseq analyses applying the algorithms RSEM and DESEQ2. Fold change in expression (FC) = 1.279; FDR (false discovery rate) = 0.009 (Analysis of tissue samples of 39 patients not suffering from AF and 11 patients with AF).

### EXAMPLES

The following Examples shall illustrate the invention. They shall, however, not be construed as limiting the scope of the invention.

### Example 1: Detection of paroxysmal AF

FABP3, NT-proBNP and hsCRP levels have been determined in plasma samples of n=60 patients with blood sampled and biomarkers assayed before open chest surgery because of CABG or valve surgery. Evidence of different types of AF, paroxysmal AF and persistent AF or SR (controls) was generated during surgery with simultaneous Endo-Epicardial High Density Activation Mapping. Patients with AF, paroxysmal AF or persistent AF and controls were matched with regard to gender, age and comorbidities.

Epicardial High Density Mapping is a different means for discrimination among subgroups of sinus rhythm, paroxysmal and persistent AF according to different conduction patterns of fibrillation waves resulting from electric activations (see Eckstein et al. and van Marion et al., both cited above).

Epicardial High Density Mapping e.g. allows for the diagnosis of paroxysmal atrial fibrillation even in the absence of an episode of Atrial Fibrillation. Thus, it allows for a reliable differentiation between subjects not suffering from AF, subjects suffering from paroxysmal AF and subjects suffering from persistent AF.

At the time of blood sampling
- patients suffering from paroxysmal AF, optional in sinus rhythm ; n=14 patients
- persistent AF patients were not in sinus rhythm; n=16 patients
- control patients were in sinus rhythm ; n=30 patients.

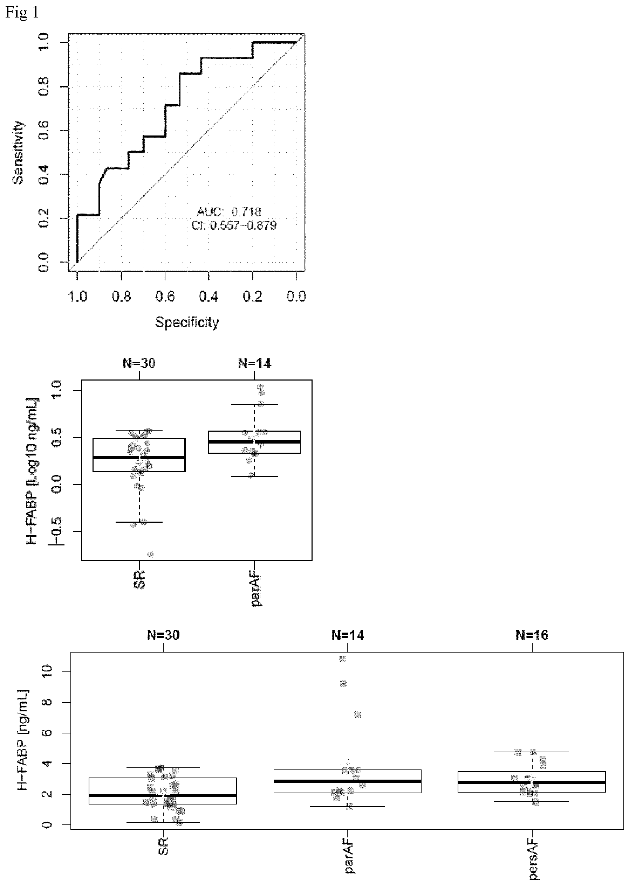

Data evaluation showed that patients with FABP3 levels (independent from other biomarkers) above a reference value (*in the study* > *2,3 ng*/*mL*) are suspected to have paroxysmal Atrial fibrillation and may benefit from anticoagulation. As shown in Figure 1 the observed AUC of FABP3 for the detection of paroxysmal AF was 0.718. The observed AUC for persistent AF was 0.718. Surprisingly it is even shown, that no progressive increase of circulating FABP3 levels could be detected from paroxysmal AF patients to persistent AF patients. The observed median circulating FABP3 values were 1.92 ng/mL in samples of 30 patients not suffering from AF, 2.84 ng/mL in samples of 14 patients suffering from paroxysmal AF; 2.75 ng/mL in samples of 16 patients with persistent AF.

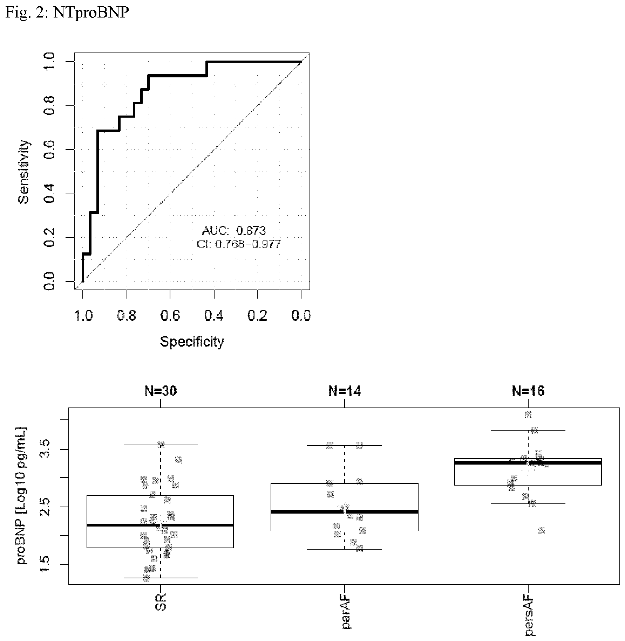

In contrast to FABP3 for NTproBNP a progressive increase of circulating levels among the subgroups of patients from paroxysmal to persistent or permanent atrial fibrillation was observed as shown in Figure 2. The observed AUC of NTproBNP for the detection of paroxysmal AF was 0.636 and 0.873 for the detection of persistent AF. Thus, in the patients analyzed herein, the biomarker FABP3 allows for an improved diagnosis of paroxysmal AF.

The observed median circulating NTproBNP values were 154.31 pg/mL in samples of 30 patients in sinus rhythm; 263.93 pg/mL in samples of 14 patients suffering from paroxysmal AF; 1829.35 pg/mL in samples of 16 patients with persistent AF.

As shown in Figure 3 the observed AUC of CRPhs for the detection of paroxysmal AF was 0.608. The observed AUC of CRPhs for the detection of persistent AF was 0.644..

It is concluded, that FABP3 shows beneficial performance versus CRPhs and may thus individually and/or in combination with NTproBNP assist in the detection of episodes of paroxysmal atrial fibrillation. Patients with FABP3 levels above a reference value (in the study > 2,3 ng/mL) in combination with elevated NTproBNP levels are suspected to have paroxysmal Atrial fibrillation and may benefit from anticoagulation.

Thus, the diagnosis of paroxysmal Atrial Fibrillation in patients presenting in sinus rhythm may be achieved with detection of enhanced levels of FABP3 alone or in combination with NT-proBNP.

### Example 2: Differential expression of FABP3 in cardiac tissue of AF patients

Differential FABP3 expression levels have been determined in myocardial tissue samples from the right atrial appendage of n=39 patients.

### RNAseq analyses

Atrial tissue was sampled during open chest surgery because of CABG or valve surgery. Evidence of AF or SR (controls) was generated during surgery with simultaneous Endo-Epicardial High Density Activation Mapping. Patients with AF and controls were matched with regard to gender, age and comorbidities.

Atrial tissue samples were prepared for
- AF patients; n=11 patients
- control patients in SR; n=39 patients.

Differential expression of FABP3 was determined in RNAseq analyses applying the algorithms RSEM and DESEQ2.

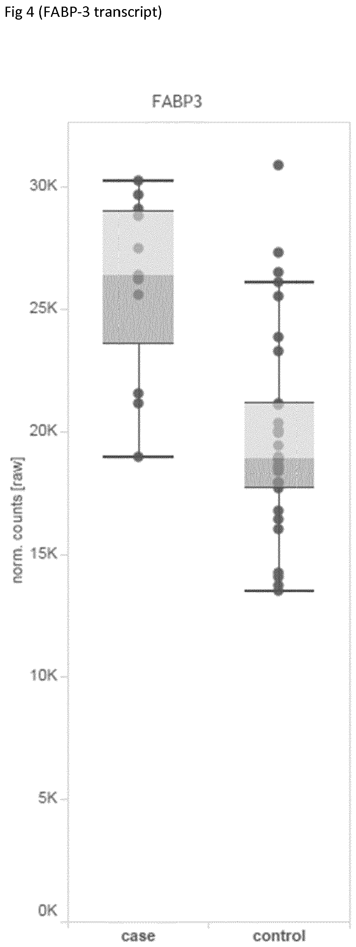

As shown in Figure 4, FABP3 expression was found to be upregulated in the analyzed atrial tissues of the 11 patients with persistent AF versus the 39 control patients.

The fold change in expression (FC) was 1.279. The FDR (false discovery rate) was 0.009. The altered expression of FABP3 was determined in the damaged end organ, the atrial tissue. FABP3 mRNA levels were compared to results of high density mapping of the atrial tissue. Elevated FABP3 mRNA levels were detected in atrial tissue samples with conduction disturbances as characterized by electrical mapping. Conductance disturbances may be caused by fat infiltration or by interstitial fibrosis. The observed differential expression of FABP3 in atrial tissue of patients suffering from atrial fibrillation supports, that FABP is released in the circulation from the myocardium, in particular from the right atrial appendage and elevated serum/plasma titers assist the detection of episodes of AF.

It is concluded, that FABP3 is released from the heart into the blood and may aid the detection of AF episodes.

## Claims

1. A method for diagnosing paroxysmal atrial fibrillation in a subject, comprising:
(a) determining the amount of FABP-3 (Fatty acid binding protein 3) in a sample from a subject suspected to suffer from paroxysmal atrial fibrillation, and
(b) comparing the amount as determined in step a) to a reference amount.

2. The method of claim 1, wherein the subject is in sinus rhythm at the time at which the sample is obtained.

3. The method of claims 1 and 2, wherein the sample is a blood, serum or plasma sample.

4. The method of any one of claims 1 to 3, wherein the subject suspected to suffer from atrial fibrillation shows or has shown at least one symptom of atrial fibrillation.

5. The method of claim 4, wherein the at least one symptom of atrial fibrillation is selected from dizziness, fainting, shortness of breath and, in particular, heart palpitations.

6. The method of any one of claims 1 to 5, wherein the subject has no known history of atrial fibrillation.

7. The method of any one of claims 1 to 6, wherein an amount of FABP-3 in the sample from the subject which is larger than the reference amount indicates that the subject suffers from paroxysmal atrial fibrillation.

8. The method of any one of claims 1 to 7, wherein an amount of FABP-3 in the sample from the subject which is lower than the reference amount indicates that the subject does not suffer from paroxysmal atrial fibrillation.

9. The method of any one of claims 1 to 8, further comprising in step a) the determination of the amount of a BNP-type peptide in a sample from the subject and in step b) the comparison of the amount of the of the BNP-type peptide to a reference amount.

10. The method of any one of claims 1 to 9, further comprising the assessment of intermittent ECG recordings obtained from said subject over a period of at least one week by using a handheld ECG device.

11. The method of any one of claims 1 to 10, wherein the subject is a human subject.

12. A method of aiding in the diagnosis of paroxysmal atrial fibrillation, said method comprising the steps of:
a) determining the amount of the biomarker FABP-3 and optionally the amount of a BNP-type peptide in a provided or obtained sample from a subject suspected to suffer from paroxysmal atrial fibrillation, and
b) providing information on the determined amount of the biomarker FABP-3 and optionally on the determined amount of the BNP-type peptide to the attending physician of the subject, thereby aiding in the diagnosis of paroxysmal atrial fibrillation in said subject.

13. Use of
i) the biomarker FABP-3 and optionally a BNP-type peptide and/or
ii) at least one detection agent that specifically binds to FABP-3, and optionally at least one detection agent that specifically binds to a BNP-type peptide,
in a sample from a subject for diagnosing paroxysmal atrial fibrillation.

14. Use of claim 13, wherein the detection agent is an antibody, or fragment thereof.

15. Use of claim 13 or 14, wherein
a) the sample is a blood, serum or plasma sample,
b) the subject is in sinus rhythm at the time at which the sample is obtained, and/or
c) the subject has no known history of atrial fibrillation.

## Patentansprüche

1. Verfahren zur Diagnose von paroxysmalem Vorhofflimmern bei einem Individuum, umfassend:
(a) Bestimmen der Menge von FABP-3 (Fettsäurebindendes Protein 3) in einer Probe von einem Individuum, bei dem ein Verdacht auf paroxysmales Vorhofflimmern besteht, und
(b) Vergleichen der in Schritt a) bestimmten Menge mit einer Referenzmenge.

2. Verfahren nach Anspruch 1, wobei das Individuum sich zum Zeitpunkt des Probennahme im Sinus-Rhythmus befindet.

3. Verfahren nach Anspruch 1 und 2, wobei es sich bei der Probe um eine Blut-, Serum-, oder Plasmaprobe handelt.

4. Verfahren nach einem der Ansprüche 1 bis 3, wobei das Individuum, bei dem ein Verdacht auf Vorhofflimmern besteht, wenigstens ein Symptom von Vorhofflimmern zeigt oder gezeigt hat.

5. Verfahren nach Anspruch 4, wobei das wenigstens eine Symptom von Vorhofflimmern ausgewählt ist aus Schwindel, Ohnmacht, Kurzatmigkeit, und, insbesondere, Herzklopfen.

6. Verfahren nach einem der Ansprüche 1 bis 5, wobei das Individuum keine bekannte Vorgeschichte von Vorhofflimmern hat.

7. Verfahren nach einem der Ansprüche 1 bis 6, wobei eine Menge von FABP-3 in der Probe von dem Individuum, die größer ist als die Referenzmenge, anzeigt, dass das Individuum an paroxysmalem Vorhofflimmern leidet.

8. Verfahren nach einem der Ansprüche 1 bis 7, wobei eine Menge von FABP-3 in der Probe von dem Individuum, die kleiner ist als die Referenzmenge, anzeigt, dass das Individuum nicht an paroxysmalem Vorhofflimmern leidet.

9. Verfahren nach einem der Ansprüche 1 bis 8, ferner umfassend in Schritt a) die Bestimmung der Menge von einem Peptid vom BNP-Typ in einer Probe von dem Individuum und in Schritt b) den Vergleich der Menge des Peptids vom BNP-Typ mit einer Referenzmenge.

10. Verfahren nach einem der Ansprüche 1 bis 9, ferner umfassend die Beurteilung von periodischen EKG-Aufnahmen, die von dem Individuum über einen Zeitraum von wenigstens einer Woche unter Verwendung eines Hand-EKG-Geräts erhalten wurden.

11. Verfahren nach einem der Ansprüche 1 bis 10, wobei es sich bei dem Individuum um einen Menschen handelt.

12. Verfahren zur Unterstützung bei der Diagnose von paroxysmalem Vorhofflimmern, wobei das Verfahren die Schritte umfasst:
a) Bestimmen der Menge des Biomarkers FABP-3 und wahlweise der Menge eines Peptids vom BNP-Typ in einer von einem Individuum, bei dem ein Verdacht auf paroxysmales Vorhofflimmern besteht, bereitgestellten oder erhaltenen Probe, und
b) Bereitstellen von Informationen bezüglich der bestimmten Menge des Biomarkers FABP-3 und wahlweise bezüglich der bestimmten Menge des Peptids vom BNP-Typ dem behandelnden Arzt des Individuums, wodurch die Diagnose von paroxysmalem Vorhofflimmern bei dem Individuum unterstützt wird.

13. Verwendung von
i) dem Biomarker FABP-3 und wahlweise einem Peptid vom BNP-Typ und/oder
ii) wenigstens einem Nachweismittel, das spezifisch an FABP-3 bindet, und wahlweise wenigstens einem Nachweismittel, das spezifisch an ein Peptid vom BNP-Typ bindet,
in einer Probe von einem Individuum zur Diagnose von paroxysmalem Vorhofflimmern.

14. Verwendung nach Anspruch 13, wobei es sich bei dem Nachweismittel um einen Antikörper, oder ein Fragment davon, handelt.

15. Verwendung nach Anspruch 13 oder 14, wobei
a) es sich bei der Probe um eine Blut-, Serum oder Plasmaprobe handelt,
b) sich das Individuum zum Zeitpunkt der Probennahme im Sinusrhythmus befindet, und/oder
c) das Individuum keine bekannte Vorgeschichte von Vorhofflimmern hat.

## Revendications

1. Procédé pour le diagnostic d'une fibrillation atriale paroxystique chez un sujet, comprenant :
(a) la détermination de la quantité de FABP-3 (protéine de liaison d'acides gras 3) dans un échantillon provenant d'un sujet suspecté de souffrir de fibrillation atriale paroxystique, et
(b) la comparaison de la quantité telle que déterminée dans l'étape a) avec une quantité de référence.

2. Procédé selon la revendication 1, le sujet étant en rythme sinusal au moment auquel l'échantillon est obtenu.

3. Procédé selon les revendications 1 et 2, l'échantillon étant un échantillon de sang, de sérum ou de plasma.

4. Procédé selon l'une quelconque des revendications 1 à 3, le sujet suspecté de souffrir de fibrillation atriale présentant ou ayant présenté au moins un symptôme de fibrillation atriale.

5. Procédé selon la revendication 4, l'au moins un symptôme de fibrillation atriale étant choisi parmi un vertige, un évanouissement, le souffle court et, en particulier, des palpitations cardiaques.

6. Procédé selon l'une quelconque des revendications 1 à 5, le sujet n'ayant pas d'antécédent de fibrillation atriale.

7. Procédé selon l'une quelconque des revendications 1 à 6, une quantité de FABP-3 dans l'échantillon provenant du sujet qui est supérieure à la quantité de référence indiquant que le sujet souffre de fibrillation atriale paroxystique.

8. Procédé selon l'une quelconque des revendications 1 à 7, une quantité de FABP-3 dans l'échantillon provenant du sujet qui est inférieure à la quantité de référence indiquant que le sujet ne souffre pas de fibrillation atriale paroxystique.

9. Procédé selon l'une quelconque des revendications 1 à 8, comprenant en outre dans l'étape a) la détermination de la quantité d'un peptide de type BNP dans un échantillon provenant du sujet et dans l'étape b) la comparaison de la quantité du peptide de type BNP avec une quantité de référence.

10. Procédé selon l'une quelconque des revendications 1 à 9, comprenant en outre l'évaluation d'enregistrements d'ECG intermittents obtenus dudit sujet sur une période d'au moins une semaine en utilisant un dispositif d'ECG portable.

11. Procédé selon l'une quelconque des revendications 1 à 10, le sujet étant un sujet humain.

12. Procédé d'aide au diagnostic d'une fibrillation atriale paroxystique, ledit procédé comprenant les étapes de :
a) détermination de la quantité du biomarqueur FABP-3 et éventuellement de la quantité d'un peptide de type BNP dans un échantillon fourni ou obtenu d'un sujet suspecté de souffrir de fibrillation atriale paroxystique, et
b) fourniture d'informations concernant la quantité déterminée du biomarqueur FABP-3 et éventuellement concernant la quantité déterminée du peptide de type BNP au médecin traitant du sujet, aidant ainsi au diagnostic d'une fibrillation atriale paroxystique chez ledit sujet.

13. Utilisation
i) du biomarqueur FABP-3 et éventuellement d'un peptide de type BNP et/ou
ii) d'au moins un agent de détection qui se lie spécifiquement à FABP-3, et éventuellement d'au moins un agent de détection qui se lie spécifiquement à un peptide de type BNP,
dans un échantillon provenant d'un sujet pour le diagnostic d'une fibrillation atriale paroxystique.

14. Utilisation selon la revendication 13, l'agent de détection étant un anticorps, ou un fragment correspondant.

15. Utilisation selon la revendication 13 ou 14,
a) l'échantillon étant un échantillon de sang, de sérum ou de plasma,
b) le sujet étant en rythme sinusal au moment auquel l'échantillon est obtenu, et/ou
c) le sujet n'ayant pas d'antécédent de fibrillation atriale.
